# EUROPÄISCHE PATENTSCHRIFT

(11) **EP 3 893 819 B1**
(45) Veröffentlichungstag und Bekanntmachung des Hinweises auf die Patenterteilung: **13.04.2022**
(21) Anmeldenummer: 19818030.9
(22) Anmeldetag: 09.12.2019
(51) Int. Cl.: A61F 5/01

(54) **ORTHESE UND VERFAHREN ZUR STEUERUNG EINER ORTHESE**
ORTHOSIS AND METHOD FOR CONTROLLING AN ORTHOSIS
ORTHÈSE ET PROCÉDÉ DE COMMANDE D'UNE ORTHÈSE

(30) Priorität: 12.12.2018 DE 102018131852
(43) Veröffentlichungstag der Anmeldung: 20.10.2021
(73) Patentinhaber: Ottobock SE & Co. KGaA, 37115 Duderstadt (DE)
(72) Erfinder: HOCHMANN, David, 48565 Steinfurt (DE); DREWITZ, Heiko, 37130 Gleichen (DE); SIEWERT, Gordon, 37083 Göttingen (DE); MÜLLER, André, 37115 Immingerode (DE); KROLL-ORYWAHL, Olaf, 37154 Northeim (DE)
(74) Vertreter: Gramm, Lins & Partner Patent- und Rechtsanwälte PartGmbB
(86) Internationale Anmeldenummer: PCT/EP2019/084225
(87) Internationale Veröffentlichungsnummer: WO 2020/120396

(56) Entgegenhaltungen:
- EP-A1- 3 301 680
- CA-A1- 2 975 606
- US-A1- 2017 319 368
- US-B2- 8 490 301

## Beschreibung

Die Erfindung betrifft eine Orthese für untere Gliedmaße mit einem Fußteil, das eine Sohle zur Abstützung eines Fußes aufweist, und zumindest einer sich davon erstreckenden Beinschiene, die sich im angelegten Zustand entlang eines Unterschenkels erstreckt und Einrichtungen zum Abstützen und/oder Festlegen der Beinschiene an einem Unterschenkel aufweist. Die Erfindung betrifft ebenfalls ein Verfahren zum Steuern einer solchen Orthese.

Orthesen sind funktionssichernde, körperanliegende, medizinische Hilfsmittel, die zur Unterstützung von eingeschränkt funktionstüchtigen Körperteilen genutzt werden, wobei insbesondere stabilisierende, mobilisierende, entlastende und korrigierende Funktionen der Orthese in dem Vordergrund stehen. Bei den sogenannten AFO (Ankle-Foot-Orthosis) handelt es sich um Orthesen, die zur Kompensation insuffizienter Unterschenkelmuskulatur nach peripheren Lähmungen sowie zur Gewährleistung einer Kniestabilität bei einer Quadriceps-Schwäche eingesetzt werden.

Die Versorgung insuffizienter Unterschenkelmuskulatur folgt heute fast ausschließlich über sogenannte Fußheberorthesen, die keinen Einfluss auf das Kniegelenk bzw. die Wadenmuskulatur haben und daher die Entstehung von Fehlstellungen als tertiäre Folge einer Beeinträchtigung nicht verhindern können. Die Versorgung bei einer Quadriceps-Schwäche findet überwiegend mit Orthesen statt, die auch das Knie überbrücken, sogenannte KAFO (Knee-Ankle-Foot-Orthosis).

Die DE 10 2004 030 570 A1 beschreibt ein Stützelement für eine Unterschenkelorthese, bei der eine Unterschenkelmanschette über das elastisch verformbare Stützelement mit einer Fußmanschette verbunden ist. Die Unterschenkelmanschette und die Fußmanschette können über Klettverschlüsse an dem Unterschenkel bzw. dem Fuß festgelegt werden. Ein Schaltkreis mit einem elektromechanischen Wandler in Gestalt eines Piezoelementes wandelt mechanische Energie beim Verformen des Stützelementes in ein elektrisches Signal um, das in einem Speicher gespeichert wird. Ein Rückstell- oder Dämpfungselement ist vorgesehen, über das in Abhängigkeit von einem Signal die Federrate des Stützelementes veränderbar ist. Die gespeicherte elektrische Energie wird bei Bedarf wieder in mechanische Energie umgewandelt, beispielsweise um die Federrate der Stützfeder zu verändern, insbesondere um das Stützelement zu versteifen.

Die WO 2005/025446 A2 beschreibt eine Orthese mit einer Sohle und einem Unterschenkelteil. Von dem Fersenbereich der Sohle bis zu einem Zehenbereich der Sohle sind unterschiedliche Dicken und Steifigkeiten vorgesehen, um eine verbesserte Unterstützung des Fußes bereitzustellen.

Die US 8,490,301 B2 betrifft eine Sohlenkonstruktion mit einer Einstellvorrichtung, mit der die Orientierung der Sohle innerhalb eines Schuhs bzw. relativ zu dem Boden verändert werden kann.

Die CA 2975606 A1 betrifft eine Stützvorrichtung oder Orthese mit Einrichtungen zur einstellbaren Steifigkeit. Dazu sind Vorsprünge beispielsweise im Sohlenbereich einer Orthese angeordnet, an denen Versteifungselemente festlegbar sind, um die gewünschte Steifigkeit einzustellen bzw. die vorhandene Steifigkeit zu verändern.

Die US 2017/319368 A1 betrifft eine Orthese mit einer Fußplatte mit integrierten Sensoren, um Positionen, Bewegungen und Ausrichtungen in einem flexiblen Vorfußbereich oder einem anderen Bereich zu erfassen. Die Orthese dient zur Datensammlung, um die Wirksamkeit einer Orthese zu ermitteln. Ebenfalls können Empfehlungen für eine geänderte Geometrie oder Steifigkeit der Orthese ausgegeben werden.

Die EP 3 301 680 A1 betrifft ein System zur Unterstützung einer Plantarflexion mit einer Unterschenkelorthese mit einem Fußteil und einer Manschette zur Befestigung an einem Schienbein, wobei das Fußteil und die Manschette mit einer den Unterschenkel an der Beinhinterseite umschließenden Feder verbunden sind. An der Unterschenkelorthese sind Sensoren zur Erfassung eines Bewegungsmusters positioniert, beispielsweise Drucksensoren, Biegesensoren, Dehnungssensoren, Bewegungssensoren oder Elektroden zur Messung einer Muskelaktivität. Die aus den Sensoren ermittelten Daten werden mit Sollwerten verglichen, bei Abweichungen wird eine entsprechende Information an den Nutzer der Unterschenkelorthese ausgegeben. EP 3 301 680 A zeigt den Oberbegriff des Anspruchs 1.

Aufgabe der vorliegenden Erfindung ist es, eine Orthese und ein Verfahren zum Steuern einer Orthese bereitzustellen, mit denen eine Anpassung an Änderungen der Einsatzbedingungen erfolgen kann, um den Tragekomfort und den Nutzen für den Patienten zu erhöhen.

Erfindungsgemäß wird diese Aufgabe durch eine Orthese mit den Merkmalen des Hauptanspruchs und ein Verfahren mit den Merkmalen des nebengeordneten Anspruchs gelöst. Vorteilhafte Ausgestaltungen und Weiterbildungen der Erfindung sind in den Unteransprüchen, der Beschreibung sowie den Figuren offenbart.

Die erfindungsgemäße Orthese für untere Gliedmaßen mit einem Fußteil, das eine Sohle zur Abstützung eines Fußes aufweist, und zumindest einer sich davon erstreckenden Beinschiene, die sich im angelegten Zustand entlang eines Unterschenkels erstreckt und Einrichtungen zum Abstützen und/oder Festlegen der Beinschiene an dem Unterschenkel aufweist, sieht vor, dass der Orthese zumindest ein Sensor zur Erfassung von Orthesenparametern zugeordnet ist, der mit einer Steuereinrichtung gekoppelt ist und dass dem Fußteil zumindest eine mit der Steuereinrichtung gekoppelte Einrichtung und/oder Werkstoffe zur Veränderung der Steifigkeit der Sohle zugeordnet sind. Über die Erfassung von Orthesenparametern über Sensoren und die Umsetzung der Orthesenparameter in einer Steuereinrichtung in Signale, mit denen die Steifigkeit der Sohle verändert wird, kann eine individuelle Anpassung der Sohle an die Umgebungsbedingungen, den Patienten und dessen Gangverhalten erfolgen. Insbesondere kann die Steifigkeit des Fersenhebels und/oder die Steifigkeit des Vorfußhebels variiert werden. Dadurch ist es möglich, eine dynamisch adaptive Fußorthese für Patienten mit einer Muskelschwäche bereitzustellen, die sich an Änderungen der Umgebungsbedingungen sowie an die individuellen Gegebenheiten eines Orthesennutzers anpassen kann. Durch die Anpassung erfolgt zudem eine kniestabilsierende Wirkung, wodurch der Patient besser und sicherer laufen kann. Die Einrichtung zur Veränderung der Steifigkeit in der Sohle weist einen Aktor zur Verlagerung eines Sohleneinsatzes, ein Piezoelement, ein magnetorheologisches Medium, ein vorgespanntes Federelement und/oder ein Thermoelement auf über die einzeln oder in Kombination einer oder mehrerer der vorgenannten Elemente miteinander die gewünschte Anpassung und Anpassbarkeit der Steifigkeit der Sohle an die jeweilige Gehsituation oder an einem Patienten vorgenommen werden kann.

Der Beinschiene kann zumindest eine mit der Steuereinrichtung gekoppelte Einreichung zur Veränderung der Steifigkeit der Beinschiene zugeordnet sein, so dass neben der Beeinflussung der Steifigkeit des Vorfußhebels und/oder des Fersenhebels auch die Steifigkeit der Beinschiene variiert werden kann, so dass beispielsweise in Abhängigkeit von dem Neigungswinkel der Sohle, der auftretenden Kräfte beim Fersenstoß oder aufgrund der Ermittlung von Positionsdaten einer Kniegelenksachse die Steifigkeit der Beinschiene aktiv verändert werden kann.

Die Änderung der Steifigkeit in der Beinschiene kann beispielsweise über eine Verlagerung eines Beinschieneneinsatzes, ein Piezoelement, ein magnetorheologisches Medium, ein vorgespanntes Federelement und/oder ein Thermoelement erfolgen, über die einzeln oder in Kombination einer oder mehrerer der vorgenannten Elemente miteinander die gewünschte Anpassung und Anpassbarkeit der Steifigkeit der Beinschiene an die jeweilige Gehsituation oder an einem Patienten vorgenommen werden kann

Zwischen der Beinschiene und dem Fußteil kann ein Gelenk angeordnet sein, dem eine Bremse und/oder eine Dämpfereinrichtung zugeordnet ist, die mit der Steuereinrichtung gekoppelt ist und die in Abhängigkeit von den erfassten Orthesenparametern aktiviert oder deaktiviert wird. Auf diese Weise kann neben einer individuellen Anpassung der Beinschiene durch eine Ausrichtung in dem Gelenk und gegebenenfalls eine Anpassung der Steifigkeit der Beinschiene zusätzlich das Widerstandsmoment um das Gelenk verändert werden.

Die Beinschiene kann als ein separates Bauteil gefertigt und gelenkig an dem Fußteil angeordnet sein, so dass eine nachträgliche Montage im Gelenkbereich erfolgen kann. Alternativ dazu können die Beinschiene und das Fußteil einstückig ausgebildet sein, wobei eine gelenkige Ausgestaltung durch eine gezielte Materialschwächung und eine Ausbildung eines sogenannten Filmscharniers realisiert werden kann.

Der Beinschiene können bei einer gelenkigen Anordnung an dem Fußteil ein Antrieb zur Einstellung der Orientierung der Beinschiene relativ zu dem Fußteil und/oder eine Feststelleinrichtung zur Fixierung der Orientierung relativ zu dem Fußteil zugeordnet sein. Dadurch ist es möglich, die Stellung des Unterschenkels relativ zur Sohle individuell einzustellen und festzulegen oder auch zu verändern, so dass eine physiologisch korrekte Ausrichtung beispielsweise während des Stehens stets gegeben ist.

Die Sensoren können im Vorderfußbereich und/oder im Fersenbereich der Sohle, an der Beinschiene oder am Übergang von der Beinschiene zu dem Fußteil angeordnet sein, um relevante Orthesenparameter zu erfassen. Ebenfalls kann zumindest ein Sensor außerhalb der Orthese angeordnet sein, beispielsweise um die Position der Knieachse zu erfassen und an die Steuereinrichtung zu übermitteln.

Die Sensoren können als Kraftsensoren, Drucksensoren, Beschleunigungssensoren, Gyroskopen, Lagesensoren und/oder Winkelsensoren ausgebildet sein.

Es können mehrere Einrichtungen und/oder Werkstoffe zur Veränderung der Steifigkeit der Sohle und/oder der Beinschiene in zueinander unterschiedlichen Orientierungen vorgesehen sein, um sowohl die Sohle als auch die Beinschiene individuell und situativ einstellbar ausgestalten zu können.

Die Sohle ist vorteilhafterweise zur Aufnahme des gesamten Fußes ausgebildet, um bei den typischen Indikationen für eine Unterschenkelorthese eine vollständige Abstützung des Fußes bereitstellen zu können, so dass der Patient eine optimale Versorgung erfahren kann.

Das erfindungsgemäße Verfahren zur Steuerung einer Orthese, wie sie oben beschrieben wurde, sieht vor, dass die Steifigkeit der Sohle und/oder der Beinschiene in Abhängigkeit von Sensorwerten, die durch die an der Orthese angeordneten Sensoren ermittelt werden, verändert wird. Dadurch kann eine Anpassung an Umgebungsbedingungen erfolgen. Durch das Tragen einer optimierten und adaptiv anpassbaren Orthese können eine negative Veränderung des Gangverhaltens und der physiologisch und bewegungstechnisch korrekten Abläufe und Zustände vermieden werden.

Ein Aspekt des Verfahrens sieht vor, dass ein Ausgangswert für eine Schwerpunklage der Kraftangriffspunkte an der Sohle und/oder eine Orientierung der Beinschiene oder der Sohle im Raum festgelegt wird und bei Überschreiten des Ausgangswertes um einen festgelegten Betrag eine Veränderung der Steifigkeit der Sohle und/oder der Beinschiene in Abhängigkeit von der durch die Sensoren ermittelten Lage des Kraftangriffspunktes und/oder der Orientierung der Sohle und/oder der Beinschiene im Raum erfolgt.

Ergänzend oder alternativ dazu kann die Orientierung der Beinschiene relativ zu dem Fußteil in Abhängigkeit von den Sensorwerten verändert werden, so dass eine kniestabilisierende und knieentlastende Orientierung der Beinschiene und damit des Unterschenkels erzielt werden kann.

Bei Anordnung eines Gelenkes zwischen der Beinschiene und dem Fußteil kann dessen rotatorischer Widerstand in Abhängigkeit von den erfassten Orthesenparametern verändert werden, wodurch sich auch eine Anpassung an die Umgebungsbedingungen oder Veränderungen im physiologischen Status ausgleichen und berücksichtigen lassen.

Nachfolgend wird ein Ausführungsbeispiel der Erfindung anhand der Figuren näher erläutert. Gleiche Bezugszeichen bezeichnen gleiche Komponenten. Es zeigen:
- Figur 1: eine schematische Ansicht einer angelegten Orthese;
- Figur 2: eine Unteransicht einer Orthese;
- Figur 3: eine Variante der Sohle;
- Figur 4: eine Sohlenausgestaltung mit Bereichen unterschiedlicher Festigkeit;
- Figur 5: eine Sohlenansicht mit einem verlagerbaren Sohleneinsatz;
- Figur 6: eine Schnittdarstellung durch ein Gelenk;
- Figur 7: eine Variante der Erfindung mit einem Hydraulikschlauch in der Sohle;
- Figur 8: eine schematische Schnittdarstellung eines Versteifungselementes;
- Figur 9: eine Orthese mit Knöchelgelenk und Dämpfern;
- Figur 10: eine Sohle mit Thermoelementen;
- Figur 11: eine Sohle mit magnetorheologischen Flüssigkeiten und Elektromagneten;
- Figur 12: eine Sohle mit zueinander verschieblichen Streifen mit unterschiedlichen Elastizitäten;
- Figur 13: eine Orthese mit einem Aktuator an einer Unterschenkelschiene und verschieblich gelagerten Versteifungselementen in der Sohle;
- Figur 14: eine Sohle mit evakuierbaren Kammern;
- Figur 15: eine Sohle mit verschieblichen Federelementen;
- Figur 16: eine Sohle mit übereinander angeordneten, verschieblichen Blattfedern<
- Figur 17: eine Variante mit Drehstäben; sowie
- Figur 18: eine Darstellung einer Orthese mit einer Bremse.

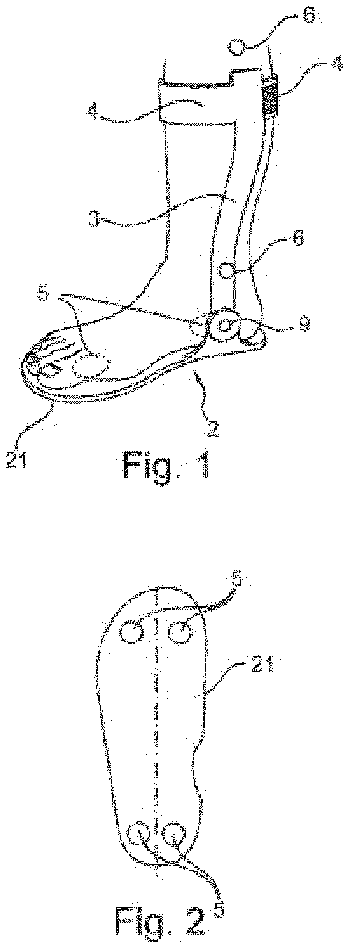

In der Figur 1 ist in einer schematischen Darstellung eine Orthese 1 für eine untere Gliedmaße mit einem Fußteil 2 dargestellt. Das Fußteil 2 weist eine Sohle 21 auf, auf der ein Fuß vollflächig abgestützt werden kann. An dem Fußteil 2 ist eine Beinschiene 3 angeordnet, die eine Einrichtung 4 in Gestalt eines Schienbeinbügels und eines Klettverschlusses zum Festlegen an einem Unterschenkel aufweist. Die Beinschiene 3 ist über ein Gelenk 9 mit dem Fußteil 2 verbunden. Über das Gelenk 9 kann eine Einstellung der Orientierung der Beinschiene 3 relativ zu dem Fußteil 2 erfolgen.

In dem Fußteil 2, der Beinschiene 3 und/oder den Einrichtungen 4 zum Abstützen der Beinschiene 3 an dem Unterschenkel können Sensoren 6 angeordnet sein, die Orthesenparameter erfassen. Es kann nur ein Sensor 6 insgesamt vorgesehen sein, ebenfalls ist es möglich, dass ein Sensor 6 jeweils in den verschiedenen Komponenten der Orthese angeordnet ist, ebenfalls können mehrere Sensoren 6 in oder an einer oder mehreren Komponenten vorhanden sein. Weiterhin kann ein Sensor 6 als Positionsgeber für die Lage einer Kompromissachse vorgesehen sein, der außerhalb der Orthese 1 an einem Patienten angebracht ist. Es können Winkelsensoren für den Winkel im oberen Sprunggelenk, Drucksensoren zur Messung der Druckbelastung in der Tibia-Anlage, Drucksensoren in der Sohle 21, Beschleunigungssensoren zur Erkennung von Gangverhalten oder Untergrundbeschaffenheiten sowie piezoelektrischen Sensoren zur Erkennung von Bodenkontakt oder Fersenabstoß vorgesehen sein.

In der Figur 2 ist eine Unteransicht des Fußteils 2 mit der Sohle 21 dargestellt. Die Sohle 21 erstreckt sich über die Gesamtlänge eines Fußes und weist beiderseits einer Längsachse Drucksensoren 5 auf, die Drücke oder Kräfte auf die Sohle 21 aufnehmen.

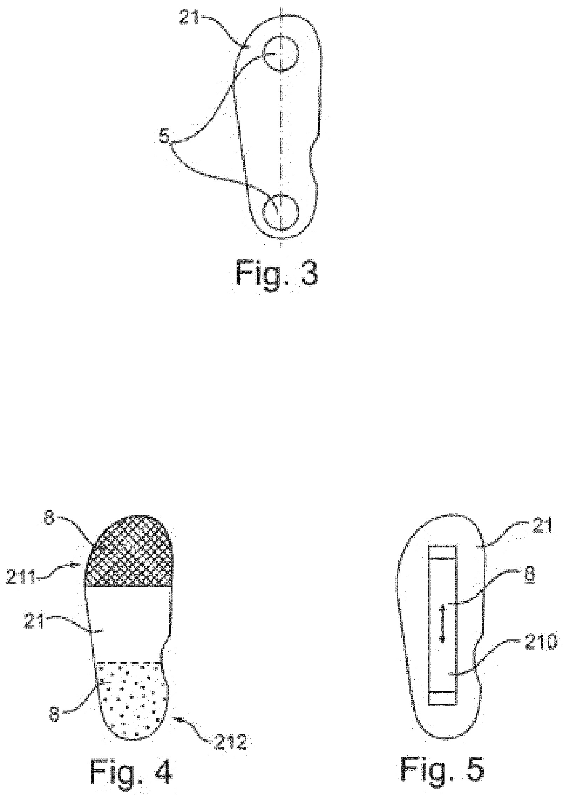

In der Figur 3 ist eine Variante der Figur 2 dargestellt, bei der die Sensoren 5 in der Sohle 21 auf der Längsachse angeordnet sind, so dass nur zwei Sensoren 5 oder Sensorbereiche in der Sohle 21 vorgesehen sind.

In der Figur 4 ist die Sohle 21 in unterschiedliche Sohlenbereiche unterteilt, nämlich in einen Fersenbereich 212 und einen davon über einen Zentralbereich getrennten Zehenbereich 211. In dem Fersenbereich 212 und in dem Zehenbereich 211 sind Werkstoffe angeordnet, die ihre mechanischen Eigenschaften durch das Anliegen äußerer Felder, beispielsweise elektrische Spannung oder durch Aktivierung magnetischer Felder, ändern. Durch das Anlegen äußerer Felder an diese Bereiche 211, 212 können die Festigkeit und Steifigkeit der jeweiligen Sohlenbereiche 211, 212 verändert werden. Dadurch ist es möglich, den effektiven Fersenhebel ebenso wie den effektiven Vorfußhebel zu verändern. Der Fersenhebel verhindert im statischen Zustand das Fallen nach hinten und kann im dynamischen Zustand, also während des Gehens, die Kniebeugung beeinflussen. Der Vorfußhebel verhindert dass Fallen nach vorne während des Stehens und kann das Abstoßen unterstützen und die Kniestreckung während des Gehens beeinflussen. Je weicher die Sohle 21 in dem jeweiligen Hebel ist, desto kürzer ist der jeweilige effektive Hebel, je steifer der jeweilige Bereich der Sohle 21 ist, desto länger ist der effektive Hebel.

Eine Variante der Erfindung ist in der Figur 5 gezeigt, bei der in der Sohle 21 ein verschieblicher Sohleneinsatz 210 als Einrichtung 8 zur Veränderung der Steifigkeit der Sohle 21 vorgesehen ist. Der Sohleneinsatz 210 kann in Abhängigkeit von den ermittelten Orthesenparametern in Richtung auf den Zehenbereich 211 oder auf den Fersenbereich 212 verschoben werden, wodurch sich der Fersenhebel und der Vorfußhebel jeweils ändern.

Eine weitere Veränderung der Orthese kann durch eine Veränderung des Aufbaus der Orthese erfolgen, indem die Orientierung der Beinschiene 3 relativ zu der Sohle 21 verändert wird. Durch den Winkel zwischen der Beinschiene 3 und der Sohle 21 wird die Kniegelenksbelastung während des Stehens und des Gehens beeinflusst, so dass eine knieschonende und gangphysiologisch günstige Stellung der Beinschiene 3 und damit des Unterschenkels relativ zu der Sohle 21 und damit zu dem vollflächig aufsitzenden Fuß erreicht werden kann.

Weiterhin ist es möglich die Steifigkeit der Beinschiene 3 durch Einrichtungen zu verändern, die den Einrichtungen 8 zur Veränderung der Steifigkeit der Sohle entsprechen. Ebenfalls kann eine Bremse oder eine Verstelleinrichtung der Beinschiene 3 zugeordnet sein, so dass in dem Gelenk 9 eine Verstellung des Winkels der Beinschiene 3 zu der Sohle 21 vorgenommen werden kann. Die Verstellung kann in Abhängigkeit der durch die Sensoren 5, 6 ermittelten Orthesenparametern erfolgen.

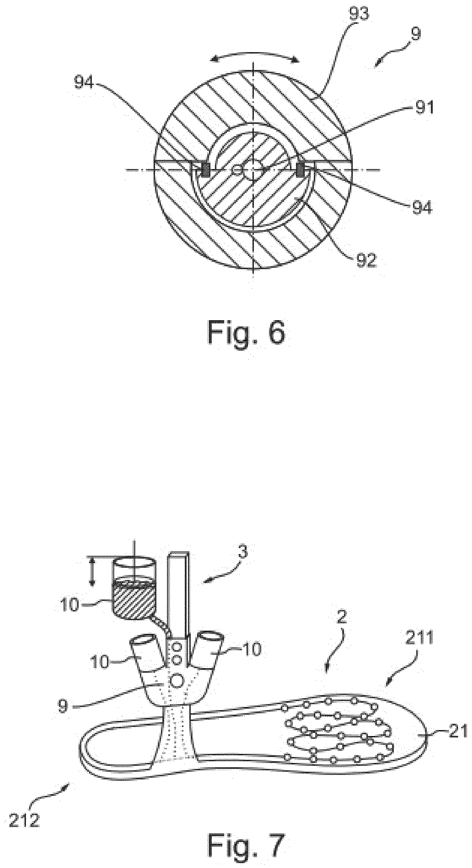

Figur 6 zeigt eine Einzeldarstellung eines Gelenkes 9 ohne Fußteil 2 und Beinschiene 3. In dem dargestellten Ausführungsbeispiel ist ein Gelenkaußenteil 93 mit einer Ausnehmung versehen, in der ein Gelenkinnenteil 92 angeordnet ist. Das Gelenkaußenteil 93 ist in dem dargestellten Ausführungsbeispiel mit der nicht dargestellten Beinschiene 3 gekoppelt. Das Gelenkinnenteil 92 ist mit dem nicht dargestellten Fußteil 2 gekoppelt, beispielsweise an dem proximalen Ende eines Fußabschnittes, das sich in proximaler Richtung von dem Fußteil 2 oder der Sohle 21 erstreckt. Das Gelenkaußenteil 93 ist um eine Schwenkachse 91, die beispielsweise auf der Höhe des natürlichen Knöchelgelenkes positioniert ist, verschwenkbar gelagert. Die Verschwenkbarkeit ist durch den Doppelpfeil dargestellt. Sowohl an dem Gelenkaußenteil 93 als auch dem Gelenkinnenteil 92 sind Vorsprünge und Anschläge ausgebildet, die einander gegenüberliegen. Zwischen den Anschlägen sind Dämpferelemente 94 angeordnet, die als Gummidämpfer ausgebildet sein können und neben einer Dämpfungswirkung auch eine Rückstellkraft gegen eine Verschwenkung ausüben, sodass das Gelenkaußenteil 93 zurück in eine Ausgangsstellung bewegt wird. Neben einer Ausgestaltung mit Anschlägen als Vorsprüngen oder Anschlagflächen kann eine gleichwirkende Ausbildung auch über eine unrunde Ausgestaltung entweder der Ausnehmung in dem Gelenkaußenteil 93 oder in der Außenkontur des Gelenkinnenteils 92 mit entsprechend angeordneten Dämpferelementen 94 erreicht werden.

Statt gummielastischer Dämpferelemente können Dämpfereinrichtungen 94 in dem Gelenk 9 eingebaut oder diesem Gelenk 9 zugeordnet werden, die mit einer nicht dargestellten Steuereinrichtung 7 gekoppelt sind. Die Dämpfereinrichtung 94 kann dann hinsichtlich ihres Widerstandes gegen eine Verschwenkbewegung eingestellt werden. Die Dämpfereinrichtung 94 kann als Hydrauliksystem ausgebildet sein, das an den jeweiligen Kontaktstellen oder Kraftübertragungsstellen zwischen dem Gelenkaußenteil 93 an dem Gelenkinnenteil 92 Kammern oder Kolben aufweist, über die Hydraulikfluid von der einen Kammer in die andere Kammer geleitet wird. Zwischen diesen Kammern kann ein Ventil oder eine einstellbare Drossel angeordnet sein, das oder die mit der Steuereinrichtung 7 gekoppelt ist und in Abhängigkeit von Sensordaten einen veränderbaren Widerstand bereitstellt. Über die Steuereinrichtung 7 kann die Dämpfereinrichtung 94 aktiviert oder deaktiviert oder hinsichtlich der gewünschten Dämpfung eingestellt werden. Als Steuerparameter oder Orthesenparameter können beispielsweise Belastungssensoren, Raumlagesensoren oder Winkelsensoren, die einen Winkel zwischen der Beinschiene 3 und dem Fußteil erfassen, vorgesehen sein.

In der Figur 7 ist eine Variante der Erfindung zeigt, bei der die Beinschiene 3 an dem Fußteil 2 befestigt ist.

In dem Fußteil 2 ist in der Sohle 21 ein Schlauchsystem eingebettet oder daran befestigt. Die Sohle 21 weist eine Flexibilität auf, die durch Veränderung des Befüllungsgrades des Schlauchsystems veränderbar ist. An der Beinschiene 3 ist ein Aktuator 10 angeordnet, der über einen Kolben Hydraulikfluid in die Hydraulikleitung hineinpumpt oder aus ihr heraussaugt. Je nach Füllgrad der Hydraulikleitung versteift sich die Sohle 21 oder wird flexibel. In der Figur 7 ist zu erkennen, dass in dem Vorderfußbereich oder Zehenbereich 211 eine größere Strecke an Hydraulikleitung als im Fersenbereich 212 verlegt ist. Dadurch ist es möglich, unterschiedliche Steifigkeiten in dem Vorderfußbereich 211 und in dem Fersenbereich 212 zu erzeugen. Das Schlauchsystem kann statt mit einem inkompressiblen Fluid über den Aktuator 10 auch mit einem kompressiblen Fluid, beispielsweise Luft, befüllt werden.

Statt oder in Ergänzung zu dem an der Beinschiene 3 angeordneten Aktuator 10 ist es möglich, dass an dem Gelenkkörper 9 zwei Aktuatoren 10 angeordnet sind, die als Pumpen wirken. Über Kolben, die durch die Verschwenkbewegung der Beinschiene 3 relativ zu dem Fußteil 2 angetrieben werden, können die Kolben bewegt und entsprechend Druck, entweder über ein Hydraulikfluid oder ein Pneumatikfluid, in dem Leitungssystem in der Sohle 21 aufgebaut oder abgebaut werden. Je nach Fülldruck versteift sich die Sohle 21 oder wird flexibler.

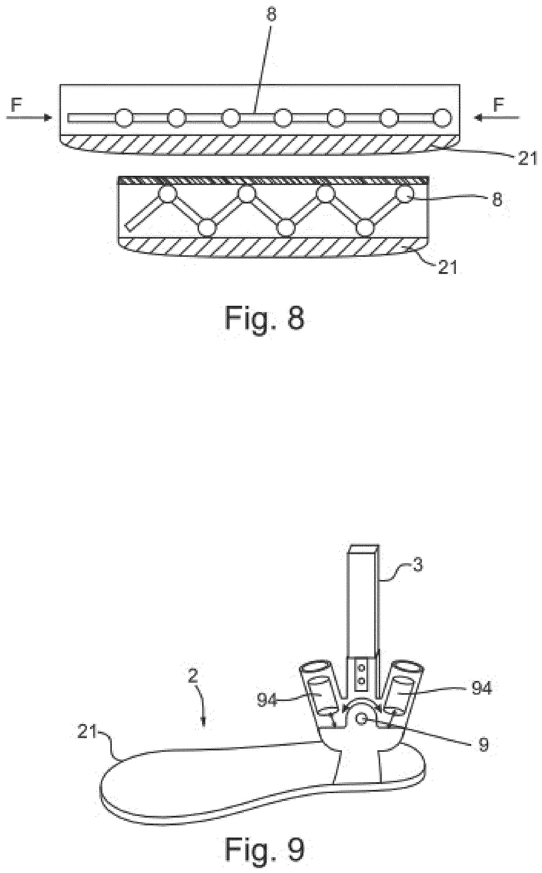

In der Figur 8 ist eine weitere Variante der Erfindung schematisch gezeigt. Die Schnittansicht zeigt zwei Zustände der Einrichtung 8 zur Veränderung der Steifigkeit, in der oberen Darstellung ist die Einrichtung 8 in Gestalt einer Gelenkkette auseinander gezogen in einem Kanal der Sohle 21 geführt. In diesem Zustand ist die Sohle 21 vergleichsweise weich und flexibel. In der unteren Darstellung der Figur 8 ist die Gelenkkette 8 zusammengeschoben, sodass sie innerhalb des Kanals an der oberen und unteren Begrenzung anliegt und dadurch eine Versteifung bewirkt. Je größer die Kraft ist, mit der die Gelenkkette zusammengeschoben wird, desto steifer ist die Sohle 21.

In der Figur 9 ist eine weitere Variante der Erfindung gezeigt. Der Aufbau entspricht im Wesentlichen dem Aufbau gemäß Figur 7, statt eines Kolbens oder eines Aktuators in dem Gelenkkörper sind zwei Dämpfer 94 in dem Gelenckörper angeordnet, die einstellbar ausgebildet sind, insbesondere in Abhängigkeit von Sensordaten, die von nicht dargestellten Sensoren ermittelt worden sind. Die Dämpfer 94 wirken einer Verschwenkung um die Schwenkachse entgegen und stellen bevorzugt gleichzeitig eine Rückstellkraft bereit, um das Gelenk 9 in eine Ausgangsstellung zurückzubewegen, wenn keine äußeren Kräfte mehr wirken. Die Dämpfer 94 können als mechanische Dämpfer, beispielsweise Elastomerelemente, hydraulische Dämpfer, pneumatische Dämpfer oder magnetorheologische Dämpfer ausgebildet sein und die Verschwenkbewegung des Fußteils 2 relativ zu der Beinschiene 3 beeinflusst. Die Sohle 21 kann zusätzlich zu den Dämpfereinrichtungen 94 in dem Gelenkkörper mit einer oder mehreren Einrichtungen zur Veränderung der Steifigkeit versehen sein.

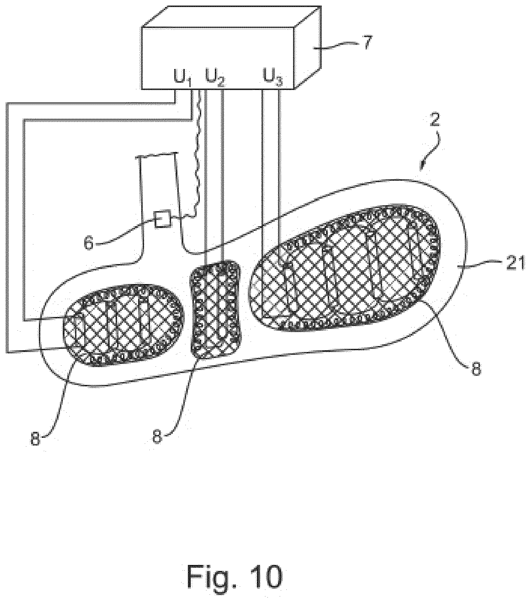

Die Figur 10 zeigt in einer Variante der Erfindung die Sohle 21 des Fußteils 2 mit Thermoelementen als Einrichtungen 8 zur Veränderung der Steifigkeit der Sohle 21. Die Thermoelemente 8 sind in der Sohle 21 integriert und wirken auf einen Materialeinsatz oder das Sohlenmaterial, der oder das in Abhängigkeit von der Temperatur seine Festigkeit und Elastizität verändert. Je wärmer das Material wird, desto weicher und flexibler wird der Materialeinsatz und damit auch die gesamte Sohle 21. Über die Steuereinrichtung 7 werden in Abhängigkeit von Sensorwerten des Sensors 6 unterschiedliche Spannungen oder unterschiedlich lange Strom durch Widerstandselemente geleitet, um eine Erwärmung zu bewirken. Die Einsätze aus einem Material mit unterschiedlichen Steifigkeiten je nach vorliegender Temperatur sind in dem dargestellten Ausführungsbeispiel in drei Bereichen angeordnet, in dem Vorderfußbereich, in dem Fersenbereich und dem Mittelfußbereich. Alle Bereiche können separat durch die Steuereinrichtung 7 angesteuert werden, um unabhängig voneinander unterschiedliche Steifigkeiten einstellen zu können. Neben einer Erwärmung ist es grundsätzlich auch möglich, über eine Abkühlung einer Erhöhung der Steifigkeit und Veränderung der Flexibilität zu erreichen.

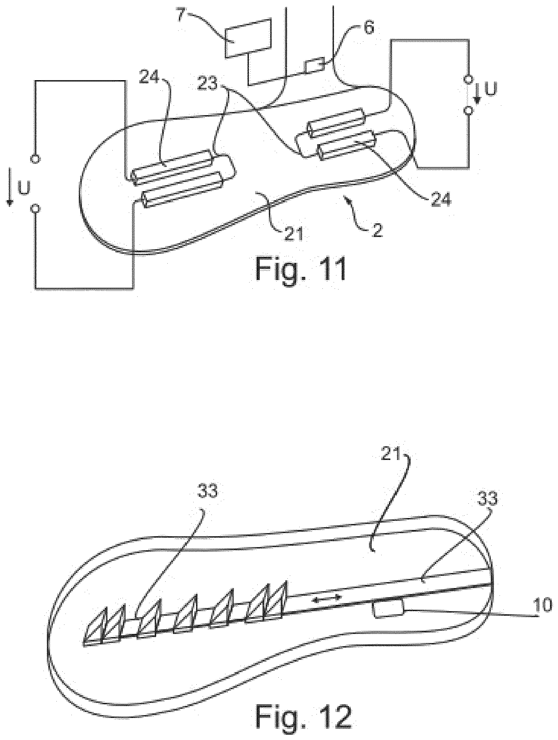

Eine weitere Variante der Erfindung ist in der Figur 11 gezeigt, bei der statt Materialeinsätzen aus einem Material mit temperaturabhängiger Steifigkeit Leitungen mit magnetorheologischer Flüssigkeit 23 in der Sohle 21 integriert sind. In Abhängigkeit von Daten eines Sensors 6 wird über die Steuereinrichtung 7 ein Magnetfeld über Elektromagneten 24 erzeugt, sodass die Partikel in der magnetorheologischen Flüssigkeit 23 hinsichtlich ihrer Viskosität verändert werden. Dadurch ist es möglich, sehr schnell die Steifigkeit in der Sohle 21 durch Veränderung des Magnetfelds zu beeinflussen.

Eine weitere Variante der Erfindung ist in der Figur 12 gezeigt, bei der in der Sohle 21 zwei Streifen 33 mit unterschiedlichen elastischen und steifen Bereichen übereinander angeordnet, die zueinander verschiebbar gelagert sind. Über einen Aktuator 9 ist es möglich, die beiden Streifen 33 in Längsersterstreckung hin und zurück zu verschieben, wie durch den Doppelpfeil angedeutet ist. An den Streifen 33 können keilartige Vorsprünge angeordnet sein, die übereinander geschoben werden können oder ineinander geschoben werden können, um unterschiedlich elastische oder flexible Bereiche auszubilden. Werden die Keile auseinander gefahren, öffnet sich ein Zwischenraum zwischen den Keilen und ermöglicht eine vergrößerte Flexibilität, werden sie zusammengeschoben, kann die Steifigkeit der Sohle 21 vergrößert werden.

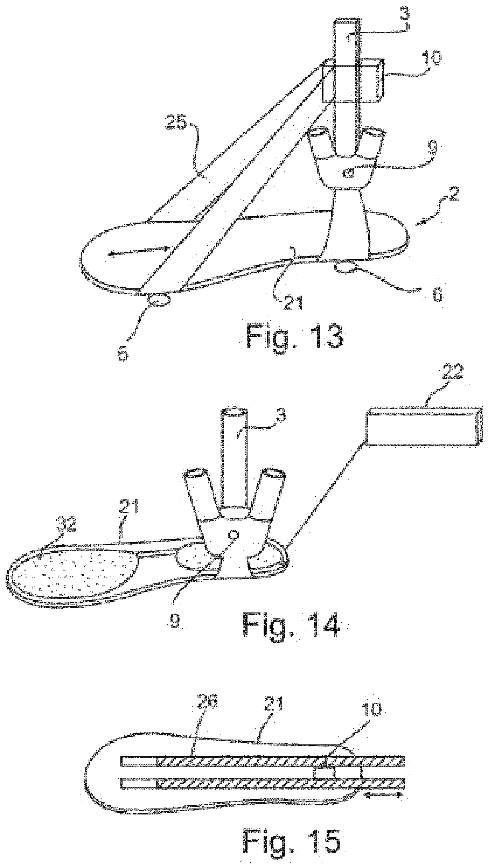

Figur 13 zeigt eine weitere Variante der Orthese mit einem an der Beinschiene angeordneten Aktuator 10, der in Abhängigkeit von Daten von Sensoren 6, die in der Sohle 21 eingearbeitet ist, verstellt wird. Der Aktuator 10 kann Bänder oder Zugelemente 25 in ihrer Länge verändern, beispielsweise durch Aufwickeln und Abwickeln oder entlang der Längserstreckung der Sohle 21 verschoben werden, wie durch den Doppelpfeil angedeutet, um so Versteifungselemente zu verschieben, Scheiben oder Streifen 33 mit unterschiedlichen elastischen Eigenschaften zueinander zu verschieben oder zu verdrehen oder um eine Vorspannung in den Bändern 25 zu erhöhen, um dadurch die Steifigkeit der Sohle 21 in Abhängigkeit von Sensordaten der Sensoren 6 zu beeinflussen.

Eine weitere Variante der Erfindung ist in der Figur 14 dargestellt, bei der in der Sohle 21 Luftkammern 32 angeordnet sind, die mit einem Granulat befüllt sein können. Über eine Vakuumpumpe 22 kann Luft aus den Luftkammern abgesaugt werden, wodurch sich das Granulat aneinander anlegt und eine Festigkeitserhöhung und dadurch eine Versteifung der Sohle 21 in dem jeweiligen Bereich erfolgt.

Eine weitere Variante der Erfindung ist in der Figur 15 dargestellt, bei der innerhalb der Sohle 2 Federstahlstreifen 26 angeordnet sind, die verschiebbar in Längserstreckung der Sohle 21 angeordnet sind. Die Streifen 26 werden durch einen Aktuator 10 in oder an der Sohle 21 verlagert und ermöglichen so eine Veränderung der Steifigkeit der Sohle in dem Bereich, in dem die Streifen 26 nicht vorhanden sind. Neben einer Ausgestaltung der Streifen 26 aus Federstahl können diese auch aus einem anderen Material ausgebildet sein.

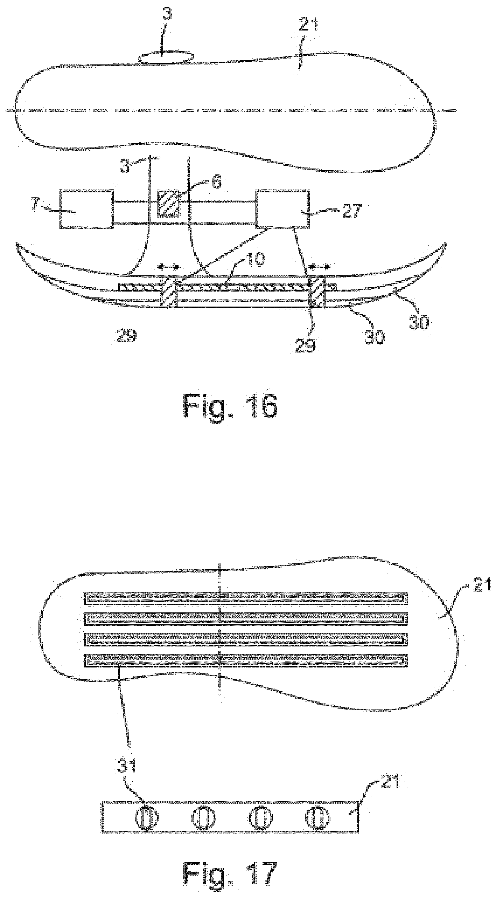

Eine weitere Variante der Erfindung ist in der Figur 16 gezeigt, bei der in der oberen Darstellung eine Untenansicht und in der unteren Darstellung eine schematische Schnittdarstellung zu erkennen sind. Die Steuereinrichtung 7 ist mit einem Sensor 6 gekoppelt, der Sensor 6 kann als Raumlagesensor oder inertial measurement unit (IMU) ausgebildet sein. Ein Energiespeicher 27, beispielsweise eine Batterie, stellt elektrische Energie bereit, um einen Schneckenantrieb als Aktuator 10 zu bewegen. Über Klemmvorrichtungen 29 sind Blattfedern 30, die übereinander angeordnet sind, mit dem Schneckenantrieb 10 gekoppelt. Der Schneckenantrieb 10 ist zweigeteilt, sodass für den Vorderfuß und die Ferse getrennt eine Veränderung der Steifigkeit durch Verschieben der einzelnen Blattfedern 30 zueinander möglich ist.

Figur 17 zeigt eine weitere Variante der Erfindung mit einer der Sohle 21 angeordneten Drehstäben 31. Die Drehstäbe 31 sind in Bohrungen oder Ausnehmungen innerhalb der Sohle 21 angeordnet und können über einen nicht dargestellten Aktuator 10 verdreht werden. Die Drehstäbe 31 sind in dem dargestellten Ausführungsbeispiel oval oder beidseitig abgeflacht ausgebildet, sodass sich unterschiedliche Flächenträgheitsmomente einstellen. Werden die Drehstäbe 31, wie in der unteren Darstellung der Figur 17 gezeigt, mit ihrem längeren Querschnitt senkrecht gestellt, also in Vertikalrichtung, erhöht sich die Steifigkeit aufgrund des größeren wirksamen Flächenträgheitsmoment. Werden die Drehstäbe 31 um 90° gedreht, verringert sich die Steifigkeit der Sohle 21 aufgrund der größeren Biegsamkeit der Drehstäbe 31 innerhalb der Sohle 21.

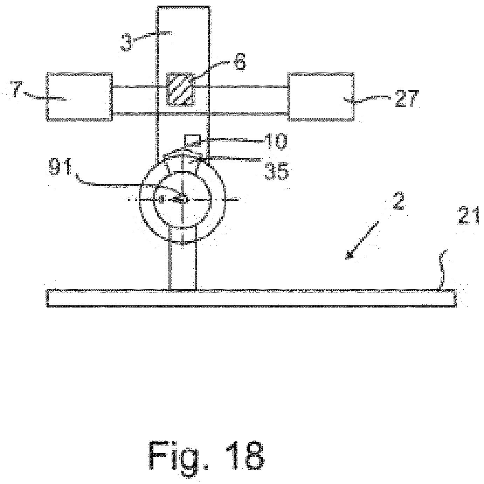

In der Figur 18 ist eine Variante der Erfindung gezeigt, bei der die Verschwenkbewegung des Fußteils 2 relativ zu der Beinschiene 3 um die Schwenkachse 91 über eine Bremse 35 beeinflusst werden kann. Die Bremseinrichtung 35 ist im dargestellten Ausführungsbeispiel als eine Scheibenbremse ausgebildet, sie kann als Backenbremse, Schlingfederbremse, elektrisch wirkende Bremse oder eine andere Bremseinrichtung ausgebildet sein. Über einen Sensor 6, der mit einer Steuerungseinheit 7 gekoppelt ist wird über Energiespeicher 27 Energie zur Aktivierung oder Deaktivierung des Aktuators 10 bereitgestellt, über den dann die Bremseinrichtung 35 aktiviert oder deaktiviert wird. Dadurch ist es möglich, die Bewegung und die Beweglichkeit zwischen der Beinschiene 3 und dem Fußteil 2 in Abhängigkeit von den über die Sensoren 6 erfassten Orthesenparameter zu beeinflussen. Weiterhin ist es vorgesehen, dass die Bremseinrichtung 35 als Motor ausgebildet ist, der deaktiviert werden kann, um eine gewählte Orientierung zwischen Beinschiene 3 und Fußteil 2 beizubehalten. Der Antrieb 35 kann in Abhängigkeit von Sensordaten die Orientierung der Beinschiene 3 zu der Sohle 21 verändern und gleichzeitig als Bremse oder als Feststelleinrichtung dienen, indem der Motor deaktiviert wird und die Bewegung behindert oder verhindert. Der Antrieb 35 kann über ein selbsthemmendes Getriebe mit dem Fußteil 2 gekoppelt sein, so dass bei inaktivierten Antrieb 35 keine Relativverlagerung möglich ist.

## Patentansprüche

1. Orthese (1) für eine untere Gliedmaße mit einem Fußteil (2), das eine Sohle (21) zur Abstützung eines Fußes aufweist, und zumindest einer sich davon erstreckenden Beinschiene (3), die sich im angelegten Zustand entlang eines Unterschenkels erstreckt und Einrichtungen (4) zum Abstützen und/oder Festlegen der Beinschiene (3) an dem Unterschenkel aufweist, wobei der Orthese (1) zumindest ein Sensor (5, 6) zur Erfassung von Orthesenparametern zugeordnet ist, der mit einer Steuereinrichtung (7) gekoppelt ist und dass dem Fußteil (2) zumindest eine mit der Steuereinrichtung (7) gekoppelte Einrichtung (8) und/oder Werkstoffe zur Veränderung der Steifigkeit der Sohle (21) zugeordnet ist, **dadurch gekennzeichnet, dass** die Einrichtung (8) zur Veränderung der Steifigkeit des Sohle (21) zumindest eine der folgenden Elemente oder Kombinationen davon aufweist: einen Aktuator (10) zur Verlagerung eines Sohleneinsatzes (210), ein Piezoelement, ein magnetorheologisches Medium (23), ein vorgespanntes Federelement (26) und/oder ein Thermoelement (8).

2. Orthese nach Anspruch 1, **dadurch gekennzeichnet, dass** der Beinschiene (3) zumindest eine mit der Steuereinrichtung (7) gekoppelte Einrichtung (8) zur Veränderung der Steifigkeit der Beinschiene (3) zugeordnet ist.

3. Orthese nach einem der voranstehenden Ansprüche, **dadurch gekennzeichnet, dass** ein Gelenk (9) zwischen der Beinschiene (3) und dem Fußteil (2) angeordnet ist und dem Gelenk (9) eine Bremse (35) und/oder eine Dämpfereinrichtung (94) zugeordnet ist, die mit der Steuereinrichtung (7) gekoppelt ist und in Abhängigkeit von den erfassten Orthesenparametern aktiviert oder deaktiviert wird.

4. Orthese nach einem der voranstehenden Ansprüche, **dadurch gekennzeichnet, dass** die Beinschiene (3) als separates Bauteil ausgebildet und gelenkig an dem Fußteil (2) angeordnet ist.

5. Orthese nach Anspruch 4, **dadurch gekennzeichnet, dass** der Beinschiene (3) ein Antrieb (35) zur Einstellung der Orientierung relativ zu dem Fußteil (2) und/oder eine Feststelleinrichtung (35) zur Fixierung der Orientierung relativ zu dem Fußteil (2) zugeordnet ist.

6. Orthese nach einem der voranstehenden Ansprüche, **dadurch gekennzeichnet, dass** Sensoren (6) im Vorfußbereich (211) und/oder im Fersenbereich (212) der Sohle (21), an der Beinschiene (3) oder am Übergang (23) von der Beinschiene (3) zu dem Fußteil (2) angeordnet sind.

7. Orthese nach einem der Ansprüche 1 bis 6, **dadurch gekennzeichnet, dass** zumindest ein Sensor (6) außerhalb der Orthese (1) angeordnet ist.

8. Orthese nach einem der voranstehenden Ansprüche, **dadurch gekennzeichnet, dass** die Sensoren (5, 6) als Kraftsensoren, Drucksensoren, Beschleunigungssensoren, Gyroskope und/oder Winkelsensoren ausgebildet sind.

9. Orthese nach einem der voranstehenden Ansprüche, **dadurch gekennzeichnet, dass** mehrere Einrichtungen (8) und/oder Werkstoffe zur Veränderung der Steifigkeit der Sohle (21) und/oder Beinschiene (3) in zueinander unterschiedlichen Orientierungen vorgesehen sind.

10. Orthese nach einem der voranstehenden Ansprüche, **dadurch gekennzeichnet, dass** die Sohle (21) zur Aufnahme eines gesamten Fußes ausgebildet ist.

11. Verfahren zur Steuerung einer Orthese (1) nach einem der voranstehenden Ansprüche, **dadurch gekennzeichnet, dass** die Steifigkeit der Sohle (21) und/oder der Beinschiene (3) in Abhängigkeit von Sensorwerten, die durch die an der Orthese (1) angeordneten Sensoren (5, 6) ermittelt wurden, verändert wird.

12. Verfahren nach Anspruch 11, **dadurch gekennzeichnet, dass** ein Ausgangswert für eine Schwerpunktlage der Kraftangriffspunkte an der Sohle (21) und/oder eine Orientierung der Beinschiene (3) oder der Sohle (21) im Raum festgelegt wird und bei Überschreiten des Ausgangswertes um einen festgelegten Betrag eine Veränderung der Steifigkeit der Sohle (21) und/oder der Beinschiene (3) in Abhängigkeit von der durch die Sensoren (5, 6) ermittelten Lage des Kraftangriffpunktes und/oder der Orientierung der Sohle (21) und/oder der Beinschiene (3) im Raum erfolgt.

13. Verfahren nach Anspruch 11 oder 12, **dadurch gekennzeichnet, dass** die Orientierung der Beinschiene (3) relativ zu dem Fußteil (2) in Abhängigkeit von den Sensorwerten verändert wird.

14. Verfahren nach einem der Ansprüche 11 bis 13, **dadurch gekennzeichnet, dass** zwischen dem Fußteil (2) und der Beinschiene (3) ein Gelenk (9) angeordnet ist, dessen rotatorischer Widerstand in Abhängigkeit von den erfassten Orthesenparametern verändert wird.

## Claims

1. An orthosis (1) for a lower limb, having a foot part (2) which has a sole (21) for supporting a foot, and having at least one leg rail (3) which extends from said foot part and which in the fitted state extends along a lower leg and which has devices (4) for supporting and/or fastening the leg rail (3) on the lower leg, wherein in that the orthosis (1) is assigned at least one sensor (5, 6) for detecting orthosis parameters, which at least one sensor is coupled to a control device (7), and in that the foot part (2) is assigned at least one device (8), which is coupled to the control device (7), and/or materials for varying the stiffness of the sole (21), **characterized in that** the device (8) for varying the stiffness of the sole (21) has at least one of the following elements, or combinations thereof: an actuator (10) for displacing a sole insert (210), a piezo element, a magnetorheological medium (23), a preloaded spring element (26), and/or a thermal element (8).

2. The orthosis as claimed in claim 1, **characterized in that** the leg rail (3) is assigned at least one device (8), which is coupled to the control device (7), for varying the stiffness of the leg rail (3).

3. The orthosis as claimed in any one of the preceding claims, **characterized in that** a joint (9) is arranged between the leg rail (3) and the foot part (2), and the joint (9) is assigned a brake (35) and/or a damper device (94) which is coupled to the control device (7) and which is activated or deactivated in a manner dependent on the detected orthosis parameters.

4. The orthosis as claimed in any one of the preceding claims, **characterized in that** the leg rail (3) is formed as a separate component and is arranged in articulated fashion on the foot part (2).

5. The orthosis as claimed in claim 4, **characterized in that** the leg rail (3) is assigned a drive (35) for setting the orientation relative to the foot part (2) and/or is assigned a fixing device (35) for fixing the orientation relative to the foot part (2).

6. The orthosis as claimed in any one of the preceding claims, **characterized in that** sensors (6) are arranged in the forefoot region (211) and/or in the heel region (212) of the sole (21), on the leg rail (3) or at the transition (23) from the leg rail (3) to the foot part (2).

7. The orthosis as claimed in any one of claims 1 to 6, **characterized in that** at least one sensor (6) is arranged outside the orthosis (1).

8. The orthosis as claimed in any one of the preceding claims, **characterized in that** the sensors (5, 6) are in the form of force sensors, pressure sensors, acceleration sensors, gyroscopes and/or angle sensors.

9. The orthosis as claimed in any one of the preceding claims, **characterized in that** multiple devices (8) and/or materials are provided for varying the stiffness of the sole (21) and/or leg rail (3) in mutually different orientations.

10. The orthosis as claimed in any one of the preceding claims, **characterized in that** the sole (21) is designed for accommodating an entire foot.

11. A method for controlling an orthosis (1) as claimed in any one of the preceding claims, **characterized in that** the stiffness of the sole (21) and/or of the leg rail (3) is varied in a manner dependent on sensor values that have been ascertained by means of the sensors (5, 6) arranged on the orthosis (1).

12. The method as claimed in claim 11, **characterized in that** a starting value for a center of gravity position of the force action points on the sole (21) and/or an orientation of the leg rail (3) or of the sole (21) in space is specified and, if the starting value is overshot by a specified amount, a variation of the stiffness of the sole (21) and/or of the leg rail (3) is performed in a manner dependent on the position, ascertained by means of the sensors (5, 6), of the force action point and/or on the orientation of the sole (21) and/or of the leg rail (3) in space.

13. The method as claimed in claim 11 or 12, **characterized in that** the orientation of the leg rail (3) relative to the foot part (2) is varied in a manner dependent on the sensor values.

14. The method as claimed in any one of claims 11 to 13, **characterized in that**, between the foot part (2) and the leg rail (3), there is arranged a joint (9), the rotational resistance of which is varied in a manner dependent on the detected orthosis parameters.

## Revendications

1. Orthèse (1) pour un membre inférieur, comprenant une partie de pied (2) qui présente une semelle (21) pour soutenir un pied, et au moins une attelle de jambe (3) qui s'étend à partir de celle-ci et qui, à l'état appliqué, s'étend le long d'un bas de jambe et qui présente des dispositifs (4) pour soutenir et/ou immobiliser l'attelle de jambe (3) sur le bas de jambe,
dans laquelle au moins un capteur (5, 6) de saisie de paramètres de l'orthèse est associé à l'orthèse (1) et est couplé à un dispositif de commande (7), et
au moins un dispositif (8) couplé au dispositif de commande (7) et/ou des matériaux destiné(s) à modifier la rigidité de la semelle (21) est/sont associé(s) à la partie de pied (2),
**caractérisée en ce que**
le dispositif (8) destiné à modifier la rigidité de la semelle (21) comprend au moins l'un des éléments suivants ou des combinaisons de ceux-ci : un actionneur (10) destiné à déplacer un insert de semelle (210), un piézo-élément, un milieu magnéto-rhéologique (23), un élément de ressort précontraint (26) et/ou un thermocouple (8).

2. Orthèse selon la revendication 1,
**caractérisée en ce qu'**au moins un dispositif (8) couplé au dispositif de commande (7) et destiné à modifier la rigidité de l'attelle de jambe (3) est associé à l'attelle de jambe (3).

3. Orthèse selon l'une des revendications précédentes,
**caractérisée en ce qu'**une articulation (9) est disposée entre l'attelle de jambe (3) et la partie de pied (2), et un frein (35) et/ou un dispositif d'amortissement (94) est associé à l'articulation (9), qui est couplé au dispositif de commande (7) et qui est activé ou désactivé en fonction des paramètres saisis de l'orthèse.

4. Orthèse selon l'une des revendications précédentes,
**caractérisée en ce que** l'attelle de jambe (3) est réalisée sous forme de composant séparé et est disposée en articulation sur la partie de pied (2).

5. Orthèse selon la revendication 4,
**caractérisée en ce qu'**un entraînement (35) destiné à régler l'orientation par rapport à la partie de pied (2) et/ou un dispositif d'immobilisation (35) destiné à fixer l'orientation par rapport à la partie de pied (2) est associé à l'attelle de jambe (3).

6. Orthèse selon l'une des revendications précédentes,
**caractérisée en ce que** des capteurs (6) sont disposés dans la zone de l'avant-pied (211) et/ou dans la zone du talon (212) de la semelle (21), sur l'attelle de jambe (3) ou à la transition (23) de l'attelle de jambe (3) vers la partie de pied (2).

7. Orthèse selon l'une des revendications 1 à 6,
**caractérisée en ce qu'**au moins un capteur (6) est disposé à l'extérieur de l'orthèse (1).

8. Orthèse selon l'une des revendications précédentes,
**caractérisée en ce que** les capteurs (5, 6) sont réalisés sous forme de capteurs de force, de capteurs de pression, de capteurs d'accélération, de gyroscopes et/ou de capteurs d'angle.

9. Orthèse selon l'une des revendications précédentes,
**caractérisée en ce que** plusieurs dispositifs (8) et/ou matériaux destinés à modifier la rigidité de la semelle (21) et/ou de l'attelle de jambe (3) sont prévus dans des orientations différentes les unes des autres.

10. Orthèse selon l'une des revendications précédentes,
**caractérisée en ce que** la semelle (21) est réalisée pour recevoir un pied entier.

11. Procédé de commande d'une orthèse (1) selon l'une des revendications précédentes,
**caractérisé en ce que** la rigidité de la semelle (21) et/ou de l'attelle de jambe (3) est modifiée en fonction de valeurs de capteur déterminées par les capteurs (5, 6) disposés sur l'orthèse (1).

12. Procédé selon la revendication 11,
**caractérisé en ce qu'**une valeur initiale est définie pour une position du centre de gravité des points d'application de la force sur la semelle (21) et/ou pour une orientation de l'attelle de jambe (3) ou de la semelle (21) dans l'espace, et, en cas de dépassement de la valeur initiale d'une valeur définie, il se produit une modification de la rigidité de la semelle (21) et/ou de l'attelle de jambe (3) en fonction de la position, déterminée par les capteurs (5, 6), du point d'application de la force et/ou de l'orientation de la semelle (21) et/ou de l'attelle de jambe (3) dans l'espace.

13. Procédé selon la revendication 11 ou 12,
**caractérisé en ce que** l'orientation de l'attelle de jambe (3) par rapport à la partie de pied (2) est modifiée en fonction des valeurs des capteurs.

14. Procédé selon l'une des revendications 11 à 13,
**caractérisé en ce qu'**une articulation (9) est disposée entre la partie de pied (2) et l'attelle de jambe (3), dont la résistance à la rotation est modifiée en fonction des paramètres saisis de l'orthèse.
